# EUROPEAN PATENT SPECIFICATION

(11) **EP 3 102 235 B1**
(45) Date of publication and mention of the grant of the patent: **27.06.2018**
(21) Application number: 15710299.7
(22) Date of filing: 09.02.2015
(51) Int. Cl.: A61K 39/215

(54) **PORCINE EPIDEMIC DIARRHEA VIRUS (PEDV) PROTEINS AND ANTIGENS**
PROTEINE UND ANTIGENE DES PORCINEN EPIDEMISCHEN DIARRHÖ VIRUS (PEDV)
PROTÉINES ET ANTIGÈNES DU VIRUS DE LA DIARRHÉE ÉPIDÉMIQUE PORCINE (VEDP)

(30) Priority: 07.02.2014 US 201461937419 P
(43) Date of publication of application: 14.12.2016
(73) Proprietor: MJ Biologics, Inc., Mankato, MN 56001 (US)
(72) Inventor: KIM, Byoung Kwan, Mankato, MN 56001 (US)
(74) Representative: J A Kemp
(86) International application number: PCT/US2015/015009
(87) International publication number: WO 2015/120378

(56) References cited:
- WO-A2-2007/006031
- J S OH: "Comparison of an enzyme-linked immunosorbent assay with serum neutralization test for serodiagnosis of porcine epidemic diarrhea virus infection", JOURNAL OF VETERINARY SCIENCE, vol. 6, no. 4, 1 January 2005 (2005-01-01) , pages 349-352, XP055188579,
- K Takamura ET AL: "Protection studies on winter dysentery caused by bovine coronavirus in cattle using antigens prepared from infected cell lysates", Canadian journal of veterinary research = Revue canadienne de recherche vétérinaire, 1 April 2000 (2000-04-01), pages 138-140, XP055188776, CANADA Retrieved from the Internet: URL:http://www.ncbi.nlm.nih.gov/pubmed/108 05255
- DAESUB SONG ET AL: "Porcine epidemic diarrhoea virus: a comprehensive review of molecular epidemiology, diagnosis, and vaccines", VIRUS GENES, vol. 44, no. 2, 22 January 2012 (2012-01-22), pages 167-175, XP035022160, ISSN: 1572-994X, DOI: 10.1007/S11262-012-0713-1
- Keizo Takamura ET AL: "Field study of bovine coronavirus vaccine enriched with hemagglutinating antigen for winter dysentery in dairy cows", Canadian journal of veterinary research = Revue canadienne de recherche vétérinaire, 1 October 2002 (2002-10-01), pages 278-281, XP055188765, Canada Retrieved from the Internet: URL:http://www.ncbi.nlm.nih.gov/pubmed/124 18784

## Description

### FIELD

This disclosure relates to the preparation and isolation of porcine epidemic diarrhea virus (PEDV) proteins and antigens. The disclosure further provides viral proteins and antigens as obtained from PEDV infected cells and in compositions comprising the proteins and antigens.

### BACKGROUND

Porcine epidemic diarrhea virus (PEDV) is a highly infectious coronavirus that infects the intestinal system of a pig, typically causing diarrhea and dehydration. While adult pigs mostly become sick and lose weight after becoming infected, the virus is often fatal to newborn piglets. Infected herds can suffer a loss of from 50% to 100% of the piglets for a four to five week period. It has been estimated that between June 2013 and March 2014 over 4 million piglets were lost to PEDV.

J. S. Oh et al, J. Vet. Sci. (2005), 6(4), 349-352 discloses a comparison of an enzyme-linked immunosorbent assay with serum neutralization test for serodiagnosis of PEDV.

### SUMMARY

This disclosure relates to proteins and antigens from the coronavirus known as PEDV. PEDV is a virus with an RNA genome, and is known to infect porcine subjects.
Disclosed herein are embodiments of a method of preparing PEDV proteins and/or antigens from cells infected with PEDV. In some embodiments, the proteins and/or antigens are harvested at an early time point after infection when the majority, or entirety, of the viral proteins and/or antigens remain associated with the infected cells. In such embodiments, the majority or entirety of PEDV encoded proteins are either within the infected cells or associated with the cell membrane of the infected cells. Under such conditions, relatively few, if any, PEDV particles are present in the extracellular environment outside the cells. In other embodiments, the proteins and/or antigens are harvested at a stage after infection when at least some of the infected cells have released replicated PEDV viral particles into the extracellular environment.

The present invention provides a method comprising:
isolating porcine epidemic diarrhea virus (PEDV)-infected cells away from cell-free PEDV viral particles in a population of cells in a culture medium; and
extracting PEDV proteins and antigens from the isolated PEDV-infected cells with a detergent-containing solution to form a solution comprising isolated PEDV proteins and antigens and the detergent, wherein the detergent-containing solution comprises a non-ionic detergent, which is poly(ethylene glycol) p-isooctyl-phenyl ether, octylphenoxypolyethoxyethanol (Nonidet P- 40), Triton X-100, or a combination thereof;
combining the solution of isolated PEDV proteins and antigens and detergent with an adjuvant to form an immunogenic composition comprising PEDV proteins and antigens, detergent and the adjuvant.

Certain embodiments of the disclosed method may include providing a population of cultured cells infected with PEDV; isolation and/or separation of the PEDV infected cells from the culture medium and/or cell-free PEDV virus in the medium; and collection of PEDV proteins and/or antigens from the isolated cells. The collection of the viral proteins and/or antigens is done by extracting or eluting them from the isolated, infected cells with a detergent-containing solution. The detergent-containing solution comprises an amount of detergent effective to extract or elute the proteins and/or antigens, such as 0.5% Triton X-100. In additional embodiments, the collecting, extracting or eluting may be for up to 24 hours, such as 2 to 15 hours or in some embodiments, from 0.2 to 5 hours. The collecting, extracting or eluting is performed at a temperature suitable for the collecting, extracting or eluting, such as from 0 °C to 25 °C, from 2 °C to 10 °C, from 2 °C to 6 °C or, in certain examples, at 4 °C. Optionally, the PEDV proteins and/or antigens produced by the method may include PEDV envelope proteins.

Also disclosed herein are isolated PEDV proteins and/or antigens prepared by the disclosed method. The viral proteins and/or antigens may include one or more PEDV envelope proteins produced by an infected cell. Compositions comprising the viral proteins and/or antigens are also contemplated. Optionally, the composition may contain one or more additional excipients, such as a pharmaceutically acceptable carrier, an adjuvant or a combination thereof.

A method of producing an immune response in a porcine subject is also disclosed. The method may include administering one or more PEDV proteins and/or antigens, prepared by the disclosed method, to the porcine subject. Optionally, the resulting immune response may be for vaccinating the porcine subject. In some aspects of the method, a composition comprising the proteins and/or antigens is administered to the subject. The method may also include one or more repeated administration(s) of the proteins and/or antigens, or the composition, to the same subject, such as 2, 3, 4 or more administrations.

A kit comprising the isolated proteins and/or antigens is disclosed herein. The kit may comprise a composition comprising the PEDV proteins and/or antigens. In aspects of the kit it is suitable for use in a method of producing an immune response in, or a method of vaccinating, a porcine subject.

The various aspects of the disclosure are contemplated for use in relation to all PEDV strains that are antigenically identical, or related to, those isolated in North America, Europe and Asia. Therefore, the disclosure may be more generally viewed as based on the protein(s) and/or antigens of any PEDV isolate, strain or subtype.

The foregoing and other objects, features, and advantages of the invention will become more apparent from the following detailed description, which proceeds with reference to the accompanying figures.

### BRIEF DESCRIPTION OF THE DRAWINGS

FIG. 1 is a Western blot of PEDV proteins from cultures of infected cells, contacted with two monoclonal antibodies.
FIG. 2 is a Western blot of PEDV proteins from cultures of infected cells over time, with a mixture of the two monoclonal antibodies used in FIG. 1.
FIG. 3 is a table of data comparing the mortality of piglets from non-vaccinated sows and sows vaccinated 3-5 days pre-farrow with an exemplary embodiment of a vaccine disclosed herein, where the non-vaccinated and vaccinated sows and litters are kept in the same room.
FIG. 4 is a table of data comparing the mortality of piglets from non-vaccinated sows and sows vaccinated 3-5 days pre-farrow with an exemplary embodiment of a vaccine disclosed herein, where the non-vaccinated and vaccinated sows and litters are kept in separate rooms.
FIG. 5 is a table of data from multiple farms, providing baseline piglet mortality data from non-vaccinated herds.

### DETAILED DESCRIPTION

### I. Definitions

The following explanations of terms and methods are provided to better describe the present disclosure and to guide those of ordinary skill in the art in the practice of the present disclosure. The singular forms "a," "an," and "the" refer to one or more than one, unless the context clearly dictates otherwise. The term "or" refers to a single element of stated alternative elements or a combination of two or more elements, unless the context clearly indicates otherwise. As used herein, "comprises" means "includes." Thus, "comprising A or B," means "including A, B, or A and B," without excluding additional elements.

Unless otherwise indicated, all numbers expressing quantities of components, molecular weights, percentages, temperatures, times, and so forth, as used in the specification or claims are to be understood as being modified by the term "about." Accordingly, unless otherwise indicated, implicitly or explicitly, the numerical parameters set forth are approximations that may depend on the desired properties sought and/or limits of detection under standard test conditions/methods. When directly and explicitly distinguishing embodiments from discussed prior art, the embodiment numbers are not approximates unless the word "about" is recited.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below. The materials, methods, and examples are illustrative only and not intended to be limiting.

PEDV protein refers to any polypeptide product encoded by the PEDV genome and/or produced as only as a result of PEDV infection or the PEDV lifecycle. Thus PEDV specific polypeptides not encoded by or expressed by a PEDV infected cell are within the scope of the term. Endogenous polypeptides encoded by a PEDV infected cell, but not expressed in the absence of PEDV infection and/or lifecycle, are not intended. However, endogenous polypeptides expressed only as a consequence of PEDV infection and/or lifecycle are within the scope of the term. The term also includes alternative forms of the polypeptides due to changes in secondary and/or tertiary structure, such as those resulting from partial or substantial protein denaturation as a non-limiting example. Thus denatured forms of the polypeptides are within the scope of the term.

PEDV antigen refers to any portion or fragment of a PEDV polypeptide that is recognized by an anti-PEDV antibody. In some cases, the portion or fragment may be a peptide with an attached moiety, such as, but not limited to, a sugar moiety, a phosphate moiety, or a lipid moiety. Alternatively, the portion or fragment may be a peptide without any attached non-peptide moieties.

An adjuvant refers to an agent that modifies the effect of another agent. As used herein an adjuvant may be added to an immunogenic composition, such as a vaccine, to modify the immune response to increase the amount of antibodies produced and/or increase the length of protection conferred by the vaccine. An adjuvant may also be added to a composition to help stabilize a formulation of proteins and/or antigens in a vaccine composition. Examples of adjuvants include, but are not limited to, inorganic compounds, such as alum, aluminum hydroxide, aluminum phosphate or calcium phosphate hydroxide; mineral oil, such as paraffin oil; bacterial products, such as killed bacteria Bordetella pertussis, Mycobacterium bovis, or toxoids; nonbacterial organics such as squalene or thimerosal; delivery systems, such as detergents (Quil A); cytokines, such as IL-1, IL-2 or IL-12; and combinations, such as Freund's complete adjuvant or Freund's incomplete adjuvant.

The pharmaceutically acceptable carriers (vehicles) useful in this disclosure are conventional. Remington: The Science and Practice of Pharmacy, The University of the Sciences in Philadelphia, Editor, Lippincott, Williams, & Wilkins, Philadelphia, PA, 21st Edition (2005), describes compositions and formulations suitable for pharmaceutical delivery of one or more therapeutic compositions and/or pharmaceutical agents.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. In some examples, the pharmaceutically acceptable carrier may be sterile to be suitable for administration to a subject (for example, by parenteral, intramuscular, or subcutaneous injection). In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

An excipient useful in this disclosure is an additive in a pharmaceutical composition. As used herein, an excipient may be incorporated within particles of a pharmaceutical composition, or it may be physically mixed with particles of a pharmaceutical composition. An excipient can be used, for example, to dilute an active agent and/or to modify properties of a pharmaceutical composition. Examples of excipients include but are not limited to polyvinylpyrrolidone (PVP), tocopheryl polyethylene glycol 1000 succinate (also known as vitamin E TPGS, or TPGS), dipalmitoyl phosphatidyl choline (DPPC), trehalose, sodium bicarbonate, glycine, sodium citrate, and lactose.

### II. Method of Extracting or Eluting PEDV Proteins and/or Antigens

The disclosure is based in part on the availability and knowledge of cell culture techniques and their use in the propagation of viruses, such as PEDV. MJ Biologics, Inc., is also the assignee of U.S. Patent Nos. 7,241,582, 7,449,296, 7,776,537 and 8,142,788which relate to the porcine reproductive and respiratory syndrome virus (PRRSV). The disclosure may be practiced by use of any suitable cell line susceptible to PEDV infection and intracellular replication *in vitro.* Thus the infected cell may be any that is capable of being productively infected by PEDV. Non-limiting examples include porcine cells, either *in vitro* or *in vivo.* One non-limiting example of cells *in vitro* is primary cells from a porcine subject that is infected with PEDV. Other non-limiting examples include simian cell lines, such as MA-104; VERO cells; BGM cells; MDCK cells and ST cells.

Infection of cells with PEDV may be at any suitable multiplicity of infection (m.o.i.), such as 0.1, 0.5 or 1, and infection of all cells in a culture is not required. In some cases, initial infection of some cells in a culture may be followed by subsequent release of infectious PEDV that infects non-infected cells in the culture. The infected cells may still be used in the practice of the disclosed methods.

After contact and infection with PEDV, the virus is allowed to intracellularly reproduce its proteins and antigens, and so replicate, for a suitable period of time. The suitable period of time may vary between different PEDV isolates, strains, and/or subtypes. In some embodiments, post-infection times ranging from 1 hour to at least 3 days, such as from 6 hours to 3 days, from 12 hours to 60 hours, or from 24 hours to 48 hours. Other post-infection times include about 12 hours, about 18 hours, about 24 hours, about 30 hours, about 36 hours, about 42 hours, about 48 hours, about 54 hours, about 60 hours, and about 66 hours. In addition to the disclosed methods, the disclosure includes a method of assessing PEDV protein production over time, after infection, to determine possible time points for isolation of infected cells and collection of viral proteins and/or antigens from the cells. This "time course" assessment after infection may be used to select a post infection time point for the preparation of viral proteins and/or antigens. The assessment is optionally performed for each PEDV isolate, strain, and/or subtype. The protein and/or antigen yields may also be compared using different PEDV isolates and different days after virus inoculation, and optimal conditions for the highest antigenic yields may be determined by comparative testing.

In some embodiments, the proteins and/or antigens harvested from PEDV infected cells may be in greater amounts than those available from PEDV particles in the culture medium. At some time points after infection, the majority, or entirety, of the replicated PEDV proteins may remain associated with the infected cells or are otherwise part of a cell associated viral component (CAVC). Thus the majority or entirety of PEDV proteins and/or antigens are either within the infected cells or associated with the cell membrane of the infected cells. Under such conditions, relatively few, if any, PEDV particles are present in the extracellular environment. The preparation of CAVC from an early time point, such as before the production of PEDV particles and/or the release thereof into the extracellular environment also has the benefit of increased safety in that no infectious viral particles are present as a contaminant.

However, in other embodiments, it was surprisingly found that harvesting the proteins and antigens at a time point after the infected cells had released replicated PEDV viral particles resulted in improved results. This was in contrast to results obtained with PRRSV.

In some embodiments, the method of preparing PEDV proteins and antigens from PEDV infected cells comprises providing a population of cells infected with PEDV; isolating the infected cells away from cell-free PEDV to form cells containing cell associated PEDV proteins and antigens; and extracting or eluting PEDV proteins and antigens from the isolated cells. In cases wherein there is no cell-free virus present, then isolating the infected cells away from cell-free PEDV may comprise isolating the infected cells from other materials that may interfere with the method, such as the culture medium used with the cells. The isolation step may be performed by any means known in the art, such as by use of centrifugation to generate a cell pellet and supernatant. The supernatant can then be removed, discarded, or otherwise separated, such as by use of a membrane filtration, to leave the cells. The extraction step is optionally performed by resuspending the cells in a buffer. The extraction or elution is performed with a detergent-containing solution, thus the buffer used to re-suspend cells may contain detergent.

In some embodiments, the method comprises using a population of cells that has been infected with PEDV for a sufficient time to produce little to undetectable amounts of infectious units per ml of supernatant, such as the culture media used with the cells. Non-limiting examples include using less than 10^{1.5} tissue culture infective dose (TCID₅₀/ml).

The detergent-containing solution comprises a non-ionic detergent, which is poly(ethylene glycol) p-isooctyl-phenyl ether, octylphenoxypolyethoxyethanol (Nonidet P- 40), Triton X-100 or a combination thereof. The detergent is used at a concentration effective for extracting or eluting PEDV proteins and/or antigens, such as a concentration of from greater than zero to 5% in solution, such as from greater than zero to 2%, 0.25% to 1%, or 0.5% in solution. In certain embodiments, the detergent is a solution of 0.5% Triton X-100. The collecting, extracting and/or eluting may be for from greater than zero to at least about 24 hours, such as from 0.1 hours to 24 hours, 0.1 hours to 15 hours, 0.2 hours to 10 hours or 0.2 hours to 5 hours, or in certain embodiments from 2 hours to 15 hours. The collecting, extracting and/or, eluting is performed at a suitable temperature. In some embodiments, the temperature is from zero to less than 30 °C, such as from 0 °C to 25 °C, from 1 °C to 20 °C, from 2 °C to 10 °C or from 2 °C to 6 °C, and in certain embodiments is 4 °C. Optionally, the viral proteins and/or antigens produced by the method include PEDV envelope proteins.

The PEDV proteins and antigens may be prepared from PEDV infected cells. In certain embodiments, the method comprises preparing the proteins and antigens from a population of the cells prepared by *in vitro* and *in vivo* methods. For the *in vitro* method, VERO cells are cultured, and the cells are harvested following an infection of PEDV.

### III. Compositions and Applications

Disclosed herein are embodiments of a composition comprising the isolated PEDV proteins and/or antigens prepared according to embodiments of the disclosed method. The composition is suitable for use for any purpose for which PEDV proteins and/or antigens are used. Non-limiting examples of applications of the proteins and/or antigens include the preparation of antibodies against the proteins/antigens; using the proteins and/or antigens as reference markers for PEDV proteins; and using the proteins and/or antigens in an immunogenic composition, such as in a vaccine formulation, optionally with a suitable carrier, adjuvant, etc., and combination thereof. The immunogenic composition may be administered to an animal to generate an immune response. Additional non-limiting examples of the compositions include those where the protein(s)/antigen(s) is/are in soluble or lyophilized (freeze dried) form.

### IV. Examples

PEDV proteins may be prepared from a PEDV strain by infecting susceptible cells *in vitro* or *in vivo,* and harvesting the infected cells at an optimal time to prepare cell-associated viral components. For *in vitro* methods, the antigen(s) may be prepared by a cell culture system or by using recombinant technologies.

In an exemplary embodiment, the cells were pelleted by centrifugation, and the supernatant was removed or discarded. The pellets may be optionally washed. PEDV proteins and antigens were extracted from the cell pellets by suspending the cell pellets in a 0.05M tris (hydroxymethyl) aminomethane 0.025M EDTA buffer containing 0.5% Triton X-100 at a volume of 5-10 times that of the packed cells. The mixture was stirred for 2-15 hours at 4 °C and then centrifuged at 10,000 g for 1 hour. The resulting supernatant comprised the PEDV proteins and antigens. Optionally, the antigen-containing solution may then undergo one or more freeze-thawing cycles, one or more of each, followed by an additional extraction cycle, to further break up intact cells and increase the efficiency of the extraction process. The freeze-thawing process also may facilitate ensuring that the antigen solution is non-infectious and allowing its use without a risk of spreading the virus.

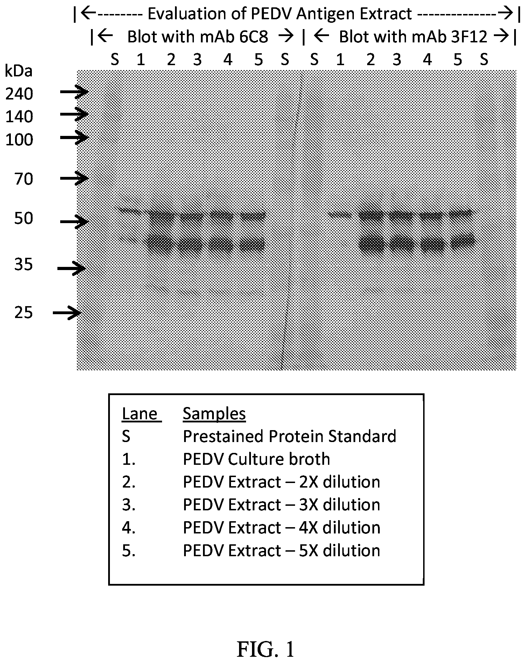

FIG. 1 provides a photograph of a Western blot of PEDV proteins. The proteins were obtained from infected cells by an exemplary embodiment of the disclosed method and were mixed with one of two monoclonal antibodies, 6C8 or 3F12, which were selected to detect certain PEDV proteins. Lanes identified as 'S' contained pre-stained protein molecular weight markers. The other lanes contained samples from a PEDV infected cell culture medium at the end of culture (lanes 1), an extract of isolated PEDV infected cells diluted 2x (lanes 2), an extract of isolated PEDV infected cells diluted 3x (lanes 3), an extract of isolated PEDV infected cells diluted 4x (lanes 4), and an extract of isolated PEDV infected cells diluted 5x (lanes 5). Surprisingly, in this example, both of the monoclonal antibodies used appeared to detect proteins with the same molecular weight.

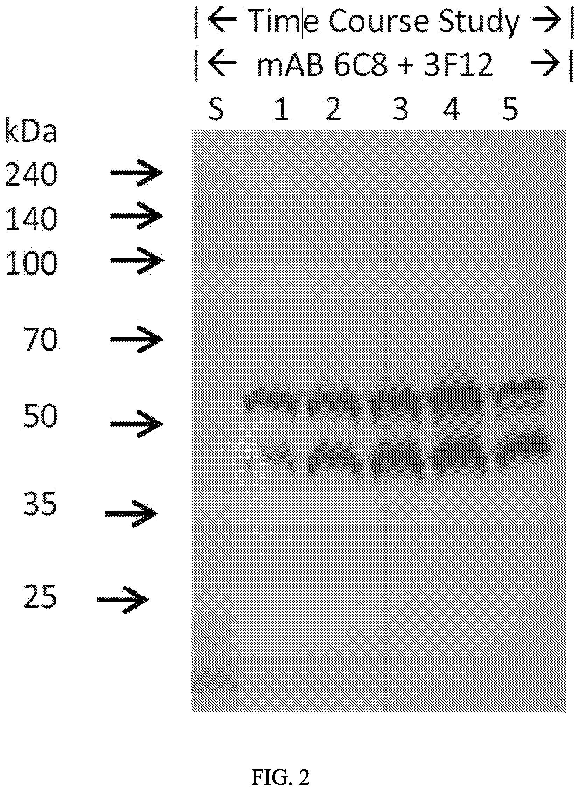

FIG. 2 provides a photograph of a Western blot of PEDV proteins from cultures of infected cells over time with a mixture of the two monoclonal antibodies used in FIG. 1. Lane S contained pre-stained protein molecular weight markers. The other lanes contained extracted samples from isolated PEDV infected cells 24 hours post infection (lane 1), 30 hours post infection (lane 2), 35 hours post infection (lane 3), 47 hours post infection (lane 4), and 52 hours post infection (lane 5).

The results from these two experiments demonstrated that the disclosed method successfully extracted proteins from cells infected with PEDV. Moreover, the Western blots illustrate that the extraction method results in a concentrated mixture of proteins compared to a culture medium, such as the culture medium of a killed virus vaccine. The concentration of proteins in the extracts or eluents may be more than twice the concentration of proteins in the culture medium, such as 4 times, 6 times, 8 times, 10 times or more than 10 times the concentration of proteins in the culture medium.

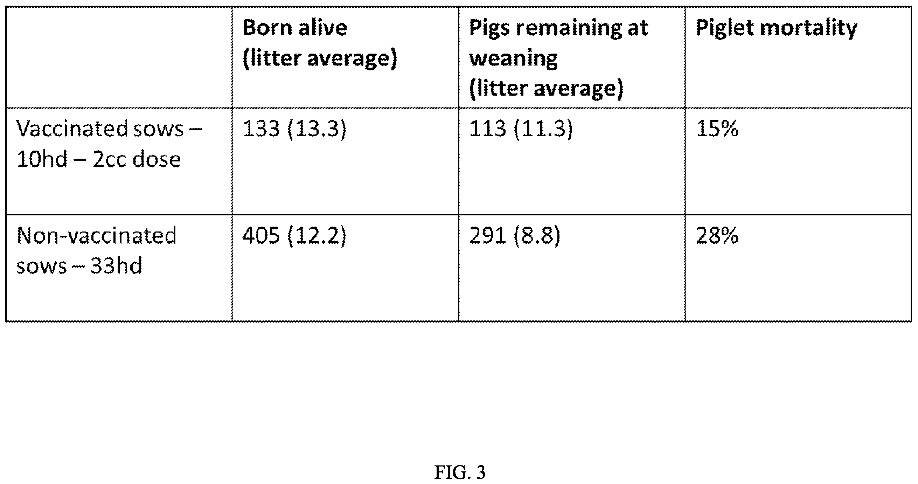

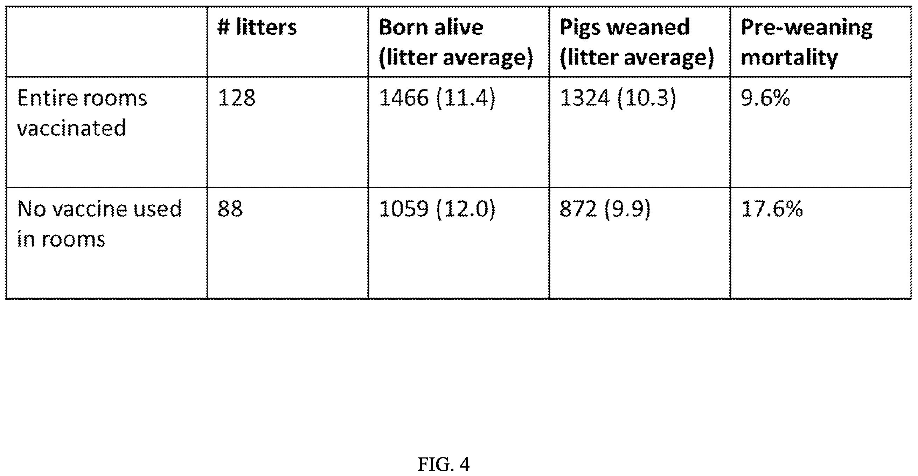

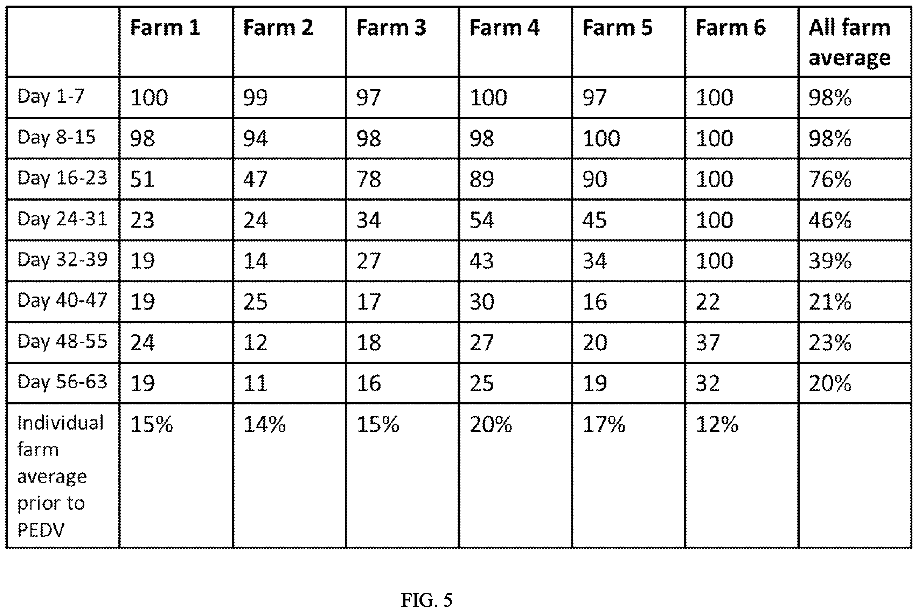

A vaccine was prepared from an exemplary antigen solution prepared by the disclosed method. The vaccine was administered to sows at 3-5 days pre-farrow in an endemic herd, 150 days post clinical break. FIG. 3 shows the data from a test where the vaccinated and non-vaccinated sows were kept in the same room. FIG. 4 shows data from a test where vaccinated and non-vaccinated sows were kept in separate rooms. The piglet mortality rates in FIGS. 3 and 4 are 15% and 9.6%, respectively, for piglets from the vaccinated sows, compared to 28% and 17.6%, respectively, for piglets from non-vaccinated sows. FIG. 5 provides baseline data from non-vaccinated herds from multiple farms, illustrating the pre-wean mortality based on days post initial whole herd PEDV virus exposure. The data in FIGS. 3-5 clearly illustrates the success of the exemplary vaccine prepared by the disclosed method.

## Claims

1. A method comprising:
isolating porcine epidemic diarrhea virus (PEDV)-infected cells away from cell-free PEDV viral particles in a population of cells in a culture medium; and
extracting PEDV proteins and antigens from the isolated PEDV-infected cells with a detergent-containing solution to form a solution comprising isolated PEDV proteins and antigens and the detergent, wherein the detergent-containing solution comprises a non-ionic detergent, which is poly(ethylene glycol) p-isooctyl-phenyl ether, octylphenoxypolyethoxyethanol or a combination thereof;
combining the solution of isolated PEDV proteins and antigens and detergent with an adjuvant to form an immunogenic composition comprising PEDV proteins and antigens, detergent and the adjuvant.

2. The method of claim 1, further comprising allowing the population of cells to incubate for a time period sufficient to result in one or more replicated PEDV viral particles being released into the culture medium.

3. The method of claim 1, wherein the detergent-containing solution comprises a detergent at a concentration of greater than zero and up to 5% in solution.

4. The method of claim 1, wherein extracting PEDV proteins and antigens comprises extracting PEDV proteins and antigens for 0.1 to 15 hours.

5. The method of claim 4, wherein extracting PEDV proteins and antigens is performed at a temperature of from 0 °C to 25 °C.

6. The method of claim 4, wherein extracting PEDV proteins and antigens comprises extracting PEDV proteins and antigens for from 0.1 hours to 15 hours at 4 °C.

7. The method of claim 1, wherein the proteins and antigens include PEDV envelope proteins.

8. The method of claim 1, wherein the solution of isolated PEDV proteins and antigens has a concentration of PEDV proteins and antigens greater than a concentration of PEDV proteins and antigens in a cell medium that contained the population of infected cells.

9. A composition comprising an immunogenic composition comprising the PEDV proteins and antigens, the detergent and adjuvant obtainable by the method of claim 1.

10. The composition of claim 9 formulated as a vaccine.

11. A composition according to claim 9 for use in a method of vaccinating a porcine subject.

12. A composition according to claim 9 for use in a method of generating an immune response in a porcine subject.

## Patentansprüche

1. Verfahren, das Folgendes umfasst:
Isolieren von Schweine-Epidemie-Diarrhoe-Virus- (Porcine Epidemic Diarrhea Virus, PEDV) infizierten Zellen von zellfreien PEDV-Viruspartikeln in einer Population von Zellen in einem Kulturmedium; und
Extrahieren von PEDV-Proteinen und Antigenen aus den isolierten PEDV-infizierten Zellen mit einer Detergens enthaltenden Lösung, um eine Lösung zu bilden, die isolierte PEDV-Proteine und Antigene und das Detergens umfasst, wobei die Detergens enthaltende Lösung ein nicht-ionisches Detergens umfasst, welches Poly(ethylenglycol)p-isooctylphenylether, Octylphenoxypolyethoxyethanol oder eine Kombination davon ist;
Kombinieren der Lösung von isolierten PEDV-Proteinen und Antigenen und Detergens mit einem Adjuvans, um eine immunogene Zusammensetzung zu bilden, die PEDV-Proteine und Antigene, Detergens und das Adjuvans umfasst.

2. Verfahren nach Anspruch 1, das ferner das Inkubieren der Population von Zellen für eine Zeitdauer umfasst, die ausreicht, um zu bewirken, dass ein oder mehrere replizierte PEDV-Viruspartikel in das Kulturmedium freigesetzt werden.

3. Verfahren nach Anspruch 1, wobei die Detergens enthaltende Lösung ein Detergens in einer Konzentration von mehr als null und bis zu 5 % in Lösung umfasst.

4. Verfahren nach Anspruch 1, wobei das Extrahieren von PEDV-Proteinen und Antigenen das Extrahieren von PEDV-Proteinen und Antigenen für 0,1 bis 15 Stunden umfasst.

5. Verfahren nach Anspruch 4, wobei das Extrahieren von PEDV-Proteinen und Antigenen bei einer Temperatur von 0 °C bis 25 °C durchgeführt wird.

6. Verfahren nach Anspruch 4, wobei das Extrahieren von PEDV-Proteinen und Antigenen das Extrahieren von PEDV-Proteinen und Antigenen für 0,1 Stunden bis 15 Stunden bei 4 °C umfasst.

7. Verfahren nach Anspruch 1, wobei die Proteine und Antigene PEDV-Hüllproteine einschließen.

8. Verfahren nach Anspruch 1, wobei die Lösung von isolierten PEDV-Proteinen und Antigenen eine Konzentration von PEDV-Proteinen und Antigenen aufweist, die größer als eine Konzentration von PEDV-Proteinen und Antigenen in einem Zellmedium ist, das die Population von infizierten Zellen enthielt.

9. Zusammensetzung, die eine immunogene Zusammensetzung umfasst, die die PEDV-Proteine und Antigene, das Detergens und das Adjuvans umfasst, die durch das Verfahren nach Anspruch 1 erhältlich sind.

10. Zusammensetzung nach Anspruch 9, die als Impfstoff formuliert ist.

11. Zusammensetzung nach Anspruch 9 zur Verwendung in einem Verfahren zur Impfung eines Schweinesubjekts.

12. Zusammensetzung nach Anspruch 9 zur Verwendung in einem Verfahren zur Erzeugung einer Immunantwort in einem Schweinesubjekt.

## Revendications

1. Procédé comprenant :
l'isolement cellules infectées par le virus de la diarrhée épidémique porcine (Porcine Epidemic Diarrhea Virus, PEDV) à partir de particules virales du PEDV acellulaires dans une population de cellules dans un milieu de culture ; et
l'extraction de protéines et d'antigènes du PEDV des cellules infectées par le PEDV isolées avec une solution contenant un détergent pour former une solution comprenant des protéines et antigènes du PEDV isolés et le détergent, la solution contenant un détergent comprenant un détergent non ionique, qui est le p-isooctyl-phényl éther de poly(éthylène glycol), l'octylphénoxypolyéthoxyéthanol ou une combinaison de ceux-ci ;
la combinaison de la solution de protéines et antigènes du PEDV isolés et de détergent avec un adjuvant pour former une composition immunogène comprenant des protéines et antigènes du PEDV, du détergent et l'adjuvant.

2. Procédé de la revendication 1, comprenant en outre le fait de permettre l'incubation de la population de cellules pendant une durée suffisante pour entraîner la libération d'au moins une particule virale du PEDV répliquée dans le milieu de culture.

3. Procédé de la revendication 1, dans lequel la solution contenant un détergent comprend un détergent à une concentration supérieure à zéro et jusqu'à 5 % en solution.

4. Procédé de la revendication 1, dans lequel l'extraction de protéines et antigènes du PEDV comprend l'extraction de protéines et antigènes du PEDV pendant 0,1 à 15 heures.

5. Procédé de la revendication 4, dans lequel l'extraction de protéines et antigènes du PEDV est effectuée à une température de 0 °C à 25 °C.

6. Procédé de la revendication 4, dans lequel l'extraction de protéines et antigènes du PEDV comprend l'extraction de protéines et antigènes du PEDV pendant 0,1 à 15 heures à 4 °C.

7. Procédé de la revendication 1, dans lequel les protéines et antigènes comprennent des protéines d'enveloppe du PEDV.

8. Procédé de la revendication 1, dans lequel la solution de protéines et d'antigènes du PEDV isolés présente une concentration en protéines et antigènes du PEDV supérieure à une concentration de protéines et antigènes du PEDV dans un milieu cellulaire qui contenait la population de cellules infectées.

9. Composition comprenant une composition immunogène comprenant les protéines et antigènes du PEDV, le détergent et l'adjuvant pouvant être obtenue par le procédé de la revendication 1.

10. Composition de la revendication 9 formulée sous la forme d'un vaccin.

11. Composition selon la revendication 9, destinée à une utilisation dans un procédé de vaccination d'un sujet porcin.

12. Composition selon la revendication 9, destinée à une utilisation dans un procédé de génération d'une réponse immunitaire chez un sujet porcin.
